(19) Europäisches Patentamt
European Patent Office
Office européen des brevets

(11) Veröffentlichungsnummer : **0 190 534**
**B1**

(12) # EUROPÄISCHE PATENTSCHRIFT

(45) Veröffentlichungstag der Patentschrift : **30.11.88**

(51) Int. Cl.[4] : **C 07 D457/02**, C 07 D457/04, C 07 D457/12

(21) Anmeldenummer : **85730167.5**

(22) Anmeldetag : **11.12.85**

(54) **Verfahren zur Herstellung von Ergolin-Derivaten.**

(30) Priorität : **13.12.84 DE 3445784**

(43) Veröffentlichungstag der Anmeldung : **13.08.86 Patentblatt 86/33**

(45) Bekanntmachung des Hinweises auf die Patenterteilung : **30.11.88 Patentblatt 88/48**

(84) Benannte Vertragsstaaten : **AT BE CH DE FR GB IT LI LU NL SE**

(56) Entgegenhaltungen :
**EP-A- 0 021 206**
**EP-A- 0 032 684**
**EP-A- 0 118 848**
**WO-A-82 /028 92**
**AT-B- 204 191**
**US-A- 3 954 772**
**JOURNAL OF THE CHEMICAL SOCIETY, PERKIN TRANSACTIONS I, Nr. 7, Juli 1984, Seiten 1401-1404, London, GB; M. KAWASE et al.: "Dehydrogenation of indolines to indoles via Azasulphonium salts or N-chloramines"**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Sauer, Gerhard, Dr.**
**Königsbacher Zeile 41A**
**D-1000 Berlin 28 (DE)**
Erfinder : **Biere, Helmut, Dr.**
**Zeltinger Strasse 15**
**D-1000 Berlin 28 (DE)**
Erfinder : **Haffer, Gregor, Dr.**
**Mörchinger Strasse 79**
**D-1000 Berlin 37 (DE)**
Erfinder : **Huth, Andreas, Dr.**
**Kirchweg 55**
**D-1000 Berlin 38 (DE)**

**EP 0 190 534 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Ergolin-Derivaten der allgemeinen Formel I

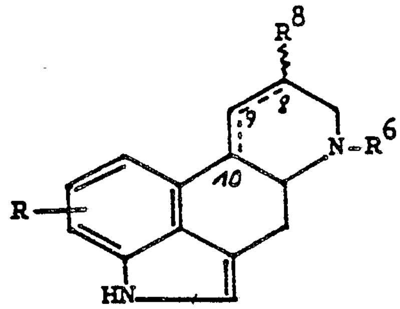

(I)

worin

$C_8$=====$C_9$ und $C_9$=====$C_{10}$ eine CC-Einfach- oder jeweils eine separate C = C-Doppelbindung darstellen und

$R^6$ $C_{1-4}$-Alkyl,

$R^8$ Methyl, Hydroxymethyl, Carbonylmethoxy, Ureido und N.N-Diethylureido in α- oder β-Stellung und

R Wasserstoff und Nitro bedeuten, dadurch gekennzeichnet, daß man ein 2.3-Dihydro-ergolin-Derivat der allgemeinen Formel II

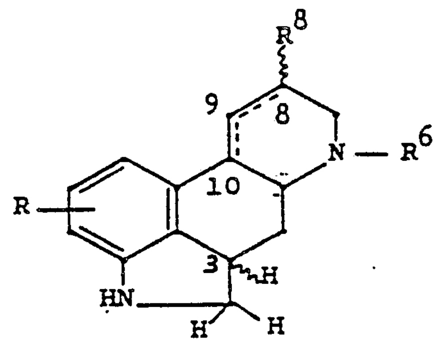

(II)

worin

$C_8$=====$C_9$ und $C_9$=====$C_{10}$ sowie $R^6$, $R^8$ und R die oben angegebene Bedeutung haben, wobei der Wasserstoff in 3-Stellung α- oder β-ständig sein kann, mit einem elektrophilen Reagenz in einem apolaren Lösungsmittel und mit einer Base bei Temperaturen von —70 bis 30 °C umsetzt.

Die erfindungsgemäß herstellbaren Verbindungen der Formel I sind entweder selbst biologisch wirksam oder können als Zwischenprodukte zur Herstellung von wertvollen Arzneimitteln verwendet werden (z. B. DE OS 33 09 493).

Der Begriff $C_{1-4}$-Alkyl in Formel I bedeutet niederes Alkyl, wie z. B. Methyl, Ethyl, n-Propyl, iso-Propyl, n-Butyl, sec.-Butyl und tert.-Butyl.

Der Substituent R kann sich in den Positionen 12, 13 und 14 der Ergolinmoleküls befinden.

Es ist bekannt, daß die 2.3-Doppelbindung in 2.3-Dihydroergonilen mit Braunstein eingeführt werden kann (z. B. US-A-3 954 772).

Die Dehydrierung mit Braunstein hat jedoch den Nachteil, daß die Ausbeuten oft gering und aufgrund der unterschiedlichen Qualitäten des Braunsteins bei größeren Ansätzen nicht reproduzierbar sind.

Aufgabe der vorliegenden Erfindung war es, ein Verfahren zur Herstellung von Ergolinen aus 2.3-Dihydro-ergolinen bereitzustellen, das in guter Ausbeute das gewünschte Ergolin liefert.

Es wurde nun überraschenderweise gefunden, daß die Dehydrierung mit elektrophilen Reagenzien in einem apolaren Lösungsmittel in Gegenwart einer Base in einfacher Weise die erfindungsgemäße Aufgabe löst.

Elektrophile Reagenzien sind beispielsweise Halogenierungsmittel wie tert.-Butylhypochlorit und N-Chlorsuccinimid, N-Bromsuccinimid, Pyrrolidon-hydroperbromid und N-Jodsuccinimid sowie Dimethylmethylthio-sulfonium-tetrafluorborat und Tosylchlorid.

Apolare Lösungsmittel sind beispielsweise Ether wie Tetrahydrofuran, Dioxan und Dimethoxyethan, chlorierte Kohlenwasserstoffe wie Methylenchlorid und Chloroform sowie aromatische Kohlenwasserstoffe wie Benzol und Toluol.

Polare Lösungsmittel wie Acetonitril, Nitromethan und Dimethylformamid sind zwar auch zur Durchführung des erfindungsgemäßen Verfahrens geeignet, jedoch nicht bevorzugt. Als Basen seien

beispielsweise Natron- und Kalilauge sowie tert. Amine wie Triethylamin und Dimethylanilin genannt.

Der Verlauf des erfindungsgemäßen Verfahrens war an sich nicht zu erwarten. Aus den Arbeiten von Kawase (M. Kawase et al., J. Chem. Soc. Perkin Trans. I (1984) 1401) ist bekannt, daß bei der Dehydrierungsreaktion von Indolinen zu Indolen mit dem Elektrophil tert.-Butylhypochlorit nur dann akzeptable Ausbeuten erhalten werden, wenn in einem ersten Schritt die N-Chlorierung in einem apolaren Losüngsmittel durchgeführt und dann in einem zweiten Schritt zur Abspaltung des Halogenwasserstoffs ein polares, aprotisches Lösungsmittel wie Dimethylformamid zugegeben wird. Bei dem erfindungsgemäßen Verfahren tritt jedoch die beim Indolin störende Halogenierung des gebildeten Indols nach dem bekannten Verfahren nicht auf.

Das erfindungsgemäße Verfahren wird zweckmäßigerweise so ausgeführt, daß man das 2.3-Dihydro-ergolin und die Base in dem apolaren Lösungsmittel löst, die Lösung gegebenenfalls abkühlt und das Elektrophil langsam zugibt. Die Reaktion ist nach 10 bis 100 Minuten beendet. Anschließend wird das Reaktionsgemisch durch Extraktion, Kristallisation und/oder Chromatographie aufgereinigt.

Die Reaktion wird bei Temperaturen im Bereich von —70 bis + 30 °C ausgeführt.

Zweckmäßig ist des weiteren ein Arbeiten unter einer Schutzgasatmosphäre.

Die Menge der zugegebenen Base beträgt 1,5-10 Moläquivalent und die Menge des Elektrophils 1,0 bis 5 Moläquivalente bezogen auf das 2.3-Dihydro-ergolin.

Die verwendeten Ausgangsmaterialien sind an sich bekannt (z. B. DE OS 33 09 493) oder können nach an sich bekannten Methoden hergestellt werden.

Die nachfolgenden Beispiele sollen das erfindungsgemäße Verfahren erläutern.

## Beispiel 1

382 mg (1 mMol) 3-(2.3β-Dihydro-6-methyl-13-nitro-8α-ergolinyl)-1.1-diethyl-harnstoff werden in 30 ml absolutem, frisch destilliertem Tetrahydrofuran unter Argon in Gegenwart von 0,5 ml Triethylamin gelöst und auf —40 °C abgekühlt. Bei dieser Temperatur gibt man 0,16 ml (1,34 mMol) t-Butylhypochlorit in 10 ml absolutem, frisch destilliertem Tetrahydrofuran gelöst tropfenweise dazu. Nach 30 Minuten Rühren bei —40 °C wird der Ansatz in 50 ml Eis eingerührt und mit 25 %iger Ammoniaklösung alkalisch gestellt. Die Extraktion erfolgt mit Methylenchlorid. Die vereinigten organischen Phasen werden über Magnesiumsulfat getrocknet und eingeengt. Das Rohprodukt wird an Silicagel mit Methylenchlorid/Methanol/Diisopropylether im Verhältnis 80 : 5 : 15 unter Druck chromatographiert (Ausbeute 351 mg) und anschließend aus Essigester/Diisopropylether kristallisiert. Dabei erhält man 272 mg 3-(6-Methyl-13-nitro-8α-ergolinyl)-1.1-diethylharnstoff (71,7 % Ausbeute).

$[\alpha]_D$ = —4,3° (0,5 % in Methanol).

## Beispiel 2

Analog Beispiel 1 wird aus 3-(2.3β-Dihydro-6-methyl-8α-ergolinyl)-1.1-diethyl-harnstoff sowie aus 3-(2.3α-Dihydro-6-methyl-8α-ergolinyl)-1.1-diethylharnstoff das Tergurid hergestellt. Ausbeute : 85 %.

$[\alpha]_D$ = + 15° (0,5 % in Chloroform) ;

aus 3-(9.10-Didehydro-2.3β-dihydro-6-methyl-14-nitro-8α-ergolinyl)-1.1-diethylharnstoff der 3-(9.10-Didehydro-6-methyl-14-nitro-8α-ergolinyl)-1.1-diethylharnstoff in 72 %iger Ausbeute,

$[\alpha]_D$ = + 336° (0,5 % in Chloroform) ;

aus 3-(9.10-Didehydro-2.3β-dihydro-6-methyl-8α-ergolinyl)-1.1-diethylharnstoff der 3-(9.10-Didehydro-6-methyl-8α-ergolinyl)-1.1-diethyl-harnstoff. Ausbeute : 45 %.

$[\alpha]_D$ = + 312° (0,4 % in Pyridin) ;

aus 1.1-Diethyl-3-(2.3α-dihydro-13-nitro-6-n-propyl-8α-ergolinyl)-harnstoff der 1.1-Diethyl-3-(13-nitro-6-n-propyl-8α-ergolinyl)-harnstoff. Ausbeute : 82 %.

$[\alpha]_D$ = + 2° (0,5 % in Chloroform) ;

aus einem Gemisch von 1.1-Diethyl-3-(2.3α-dihydro-6-n-propyl-8α-ergolinyl)-harnstoff und 1.1-Diethyl-3-(2.3β-dihydro-6-n-propyl-8α-ergolinyl)-harnstoff der 1.1-Diethyl-3-(6-n-propyl-8α-ergolinyl)-harnstoff. Ausbeute : 71 %.

$[\alpha]_D$ = + 38° (0,5 % in Pyridin) ;

aus 2.3β-Dihydro-6-methyl-8β-ergolin-carbonsäuremethylester der 6-Methyl-ergolin-8β-carbonsäuremethylester. Ausbeute : 70 %.

$[\alpha]_D$ = —100° (0,5 % in Pyridin) und

aus 3-(9.10-Didehydro-2.3β-dihydro-6-methyl-12-nitro-8α-ergolinyl)-1.1-diethyl-harnstoff der 3-(9.10-Didehydro-6-methyl-12-nitro-8α-ergolinyl)-1.1-diethylharnstoff in 67 %iger Ausbeute.

$[\alpha]_D$ = —408° (0,5 % in Chloroform).

Beispiel 3

34 mg (0,1 mMol) 3-(2.3β-Dihydro-6-methyl-8α-ergolinyl)-1.1-diethyl-harnstoff werden in 3 ml absolutem, frisch destilliertem Tetrahydrofuran unter Argon in Gegenwart von 0,05 ml Triethylamin gelöst und auf —40 °C abgekühlt. Bei dieser Temperatur gibt man 60 mg (0,12 mMol) Pyrrolidon-hydrotribromid dazu. Anschließend läßt man 20 Minuten bei —20 °C rühren. Dann wird das Reaktionsgemisch in 10 ml Eis eingerührt und mit 25 %iger Ammoniaklösung alkalisch eingestellt. Die organische Phase wird mit Methylenchlorid extrahiert. Die vereinigten organischen Phasen werden über Magnesiumsulfat getrocknet und eingeengt. Man erhält 1.1-Diethyl-3-(6-methyl-8α-ergolinyl)-harnstoff in 60 %iger Ausbeute.

$[\alpha]_D = +15°$ (0,5 % in Chloroform).

Beispiel 4

Zu 38 mg (0,1 mMol) 3-(2.3β-Dihydro-6-methyl-13-nitro-8α-ergolinyl)-1.1-diethyl-harnstoff in 2 ml absolutem, frisch destilliertem Tetrahydrofuran gibt man unter Argon 47 mg (0,24 mMol) Dimethylmethylthiosulfonium-fluoroborat und rührt 20 Minuten bei Raumtemperatur. Zur Aufarbeitung gießt man den Ansatz auf 10 ml Eis und alkalisiert mit 25 %iger Ammoniaklösung. Die organische Phase wird mit Methylenchlorid mehrfach extrahiert. Die Extrakte werden über Magnesiumsulfat getrocknet und eingeengt. Das Rohprodukt reinigt man über eine präparative Kieselgelplatte mit Methylenchlorid/Methanol 9 : 1 als Laufmittel, wobei 28 mg 3-(2.3β-Dihydro-6-methyl-1-methylthio-13-nitro-8α-ergolinyl)-1.1-diethylharnstoff erhalten werden.

25 mg des zuvor erhaltenen 3-(2.3β-Dihydro-6-methyl-1-methylthio-13-nitro-8α-ergolinyl)-1.1-diethylharnstoffs werden in 0,5 ml Methanol mit 0,5 ml 7n Kalilauge unter Argon bei Raumtemperatur gerührt. Nach 2 Stunden wird der Ansatz unter Eiskühlung mit gesättigter Kochsalzlösung versetzt und mit Methylenchlorid extrahiert. Die organischen Phasen werden mit gesättigter Kochsalzlösung und Wasser neutral gewaschen und über Magnesiumsulfat getrocknet. Die organischen Phasen werden eingeengt. Die Ausbeute an 3-(6-Methyl-13-nitro-8α-ergolinyl)-1.1-diethyl-harnstoff ist quantitativ.

$[\alpha]_D = -4°$ (0,5 % in Chloroform).

**Patentansprüche**

1. Verfahren zur Herstellung von Ergolin-Derivaten der allgemeinen Formel I

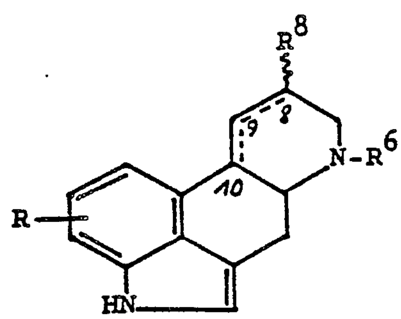

(I)

worin

$C_8\text{====}C_9$ und $C_9\text{====}C_{10}$ eine CC-Einfach- oder jeweils eine separate C = C-Doppelbindung darstellen und

$R^6$ $C_{1-4}$-Alkyl,

$R^8$ Methyl, Hydroxymethyl, Carbonylmethoxy, Ureido und N.N-Diethylureido in α- oder β-Stellung und

R Wasserstoff und Nitro bedeuten,

dadurch gekennzeichnet, daß man ein 2.3-Dihydro-ergolin-Derivat der allgemeinen Formel II

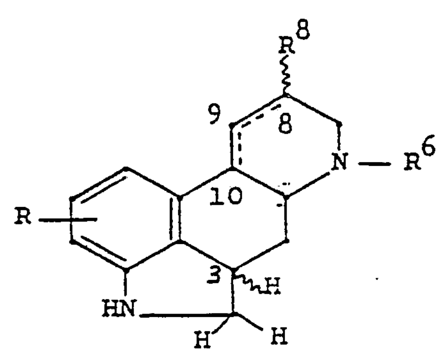

(II)

worin

$C_8$═══$C_9$ und $C_9$═══$C_{10}$ sowie $R^6$, $R^8$ und R die oben angegebene Bedeutung haben, wobei der Wasserstoff in 3-Stellung α- oder β-ständig sein kann, mit einem elektrophilen Reagenz in einem apolaren Lösungsmittel und mit einer Base bei Temperaturen von —70 bis 30 °C umsetzt.

2. Verfahren nach Anspruch 1, dadurch gekennzeichnet, daß man als elektrophiles Reagentz tert.-Butylhypochlorit verwendet.

3. Verfahren nach Anspruch 1, dadurch gekennzeichnet, daß man als apolares Lösungsmittel Tetrahydrofuran verwendet.

4. Verfahren nach Anspruch 1, dadurch gekennzeichnet, daß man als Base Triethylamin verwendet.

5. Verfahren nach Anspruch 1, dadurch gekennzeichnet, daß man als elektrophiles Reagenz Dimethyl-methylthiosulfoniumfluoroborat verwendet und anschließend mit Alkalihydroxid eliminiert.

**Claims**

1. A process for the production of ergoline derivatives of the general formula I

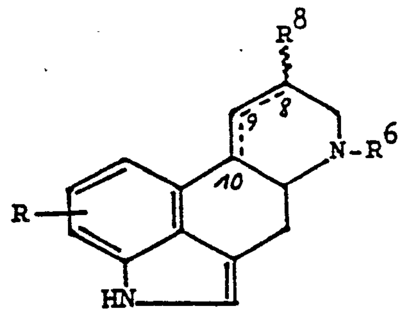

(I)

wherein

$C_8$═══$C_9$ and $C_9$═══$C_{10}$ are each a CC-single bond or each is a separate C = C-double bond,

$R^6$ is $C_{1-4}$alkyl,

$R^8$ is methyl, hydroxymethyl, carbonylmethoxy, ureido or N,N-diethylureido, each in the α- or β-configuration,

and

R is hydrogen or nitro,

characterised in that a 2,3-dihydroergoline derivative of the general formula II

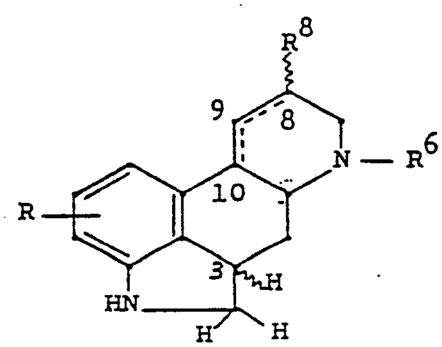

(II)

wherein $C_8$═══$C_9$ and $C_9$═══$C_{10}$ and also $R^6$, $R^8$ and R have the meanings indicated above, it being possible for the hydrogen in the 3-position to have the α- or β-configuration, is reacted with an electrophilic reagent in an apolar solvent and with a base at temperatures of from —70 to 30 °C.

2. A process according to claim 1, characterised in that tert.-butyl hypochlorite is used as the electrophilic reagent.

3. A process according to claim 1, characterised in that tetrahydrofuran is used as the apolar solvent.

4. A process according to claim 1, characterised in that triethylamine is used as the base.

5. A process according to claim 1, characterised in that dimethylmethylthiosulfonium fluoroborate is used as the electrophilic reagent and then elimination with alkali metal hydroxide is carried out.

**Revendications**

1. Procédé de préparation de dérivés d'ergoline de formule générale I ci-dessous :

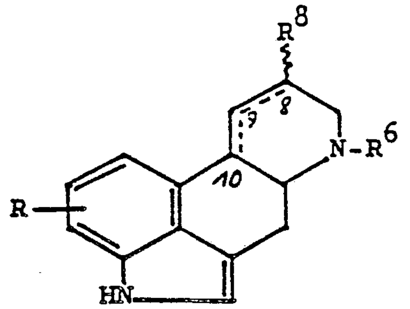

(I)

formule dans laquelle :

les liaisons $C_8$====$C_9$ et $C_9$====$C_{10}$ représentent une liaison CC simple ou chacune une double-liaison C = C séparée,

$R^6$ désigne un alkyle en $C_{1-4}$,

$R^8$ un groupe méthyle, hydroxyméthyle, méthoxycarbonyle, uréido ou N,N-diéthyluréido à la position α ou β, et

R l'hydrogène ou le groupe nitro,

procédé caractérisé en ce que l'on fait réagir une 2,3-dihydro-ergoline de formule générale II

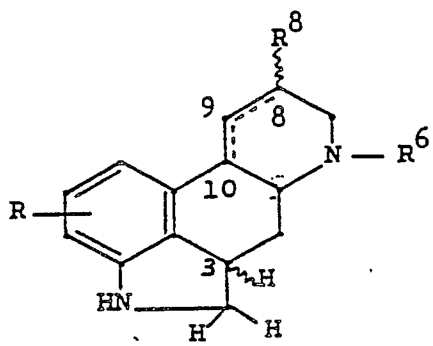

(II)

les divers symboles ayant les mêmes significations que dans la formule I et l'hydrogène à la position 3 pouvant être en α ou en β, avec un réactif électrophile dans un solvant apolaire et avec une base, à une température comprise entre —70 et 30 °C.

2. Procédé selon la revendication 1 caractérisé en ce que l'on utilise l'hypochlorite de butyle tertiaire comme réactif électrophile.

3. Procédé selon la revendication 1 caractérisé en ce que l'on utilise le tétrahydrofuranne comme solvant apolaire.

4. Procédé selon la revendication 1 caractérisé en ce que l'on utilise la triéthylamine comme base.

5. Procédé selon la revendication 1 caractérisé en ce que l'on utilise comme réactif électrophile le fluoroborate de diméthyl-méthylthiosulfonium, que l'on élimine ensuite avec un hydroxyde de métal alcalin.